(19) Europäisches Patentamt
European Patent Office
Office européen des brevets

(11) **EP 3 278 269 B1**

(12) **FASCICULE DE BREVET EUROPEEN**

(45) Date de publication et mention
de la délivrance du brevet:
**29.05.2024   Bulletin 2024/22**

(21) Numéro de dépôt: **16717851.6**

(22) Date de dépôt: **01.04.2016**

(51) Classification Internationale des Brevets (IPC):
*A61B 5/00* (2006.01)   *A61B 5/055* (2006.01)
*A61B 5/35* (2021.01)   *A61B 5/366* (2021.01)
*G06T 7/00* (2017.01)   *G06T 7/11* (2017.01)
*G06T 7/187* (2017.01)   *G06V 20/64* (2022.01)

(52) Classification Coopérative des Brevets (CPC):
**G06T 7/0012; A61B 5/0044; A61B 5/055;
A61B 5/35; A61B 5/366; A61B 5/489; G06T 7/11;
G06T 7/187; G06V 20/64;** G06F 2218/16;
G06F 2218/22; G06T 2207/20152;
G06T 2207/30048; G06V 2201/031

(86) Numéro de dépôt international:
**PCT/EP2016/057237**

(87) Numéro de publication internationale:
**WO 2016/156578 (06.10.2016 Gazette 2016/40)**

(54) **PROCÉDÉ ET SYSTÈME D'IDENTIFICATION D'UN ISTHME DANS UNE CARTOGRAPHIE TRIDIMENSIONNELLE**

VERFAHREN UND SYSTEM ZUR IDENTIFIZIERUNG EINES ISTHMUS AUF EINER DREIDIMENSIONALEN KARTE

METHOD AND SYSTEM FOR IDENTIFYING AN ISTHMUS IN A THREE-DIMENSIONAL MAP

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **03.04.2015   FR 1552901**

(43) Date de publication de la demande:
**07.02.2018   Bulletin 2018/06**

(73) Titulaires:
• **Université de Lorraine
54052 Nancy Cedex (FR)**
• **Centre Hospitalier Régional de Nancy
54035 Nancy Cedex (FR)**

(72) Inventeur: **DE CHILLOU, Christian
57155 Marly (FR)**

(74) Mandataire: **IPAZ
Bâtiment Platon
Parc Les Algorithmes
91190 Saint-Aubin (FR)**

(56) Documents cités:
• **T. DICKFELD ET AL: "MRI-Guided Ventricular
Tachycardia Ablation: Integration of Late
Gadolinium-Enhanced 3D Scar in Patients With
Implantable Cardioverter-Defibrillators",
CIRCULATION. ARRHYTHMIA AND
ELECTROPHYSIOLOGY, vol. 4, no. 2, 26 janvier
2011 (2011-01-26), pages 172-184, XP055243295,
United States ISSN: 1941-3149, DOI:
10.1161/CIRCEP.110.958744**
• **Pr Christian De Chillou: "Catheter ablation of
post-infarct VT Background", Institut Lorrain du
Coeur et des Vaisseaux - University Hospital
Nancy - France, 30 janvier 2014 (2014-01-30),
pages 1-97, XP055243384, Extrait de l'Internet:
URL:http://sfcardio.fr/sites/default/files
/pdf/Chillou-a565.pdf [extrait le 2016-01-20]**
• **W. G. STEVENSON ET AL: "Catheter Ablation for
Ventricular Tachycardia", CIRCULATION, vol.
115, no. 21, 14 mai 2007 (2007-05-14) , pages
2750-2760, XP055243396, US ISSN: 0009-7322,
DOI: 10.1161/CIRCULATIONAHA.106.655720**

- CHRISTIAN DE CHILLOU ET AL: "Showing Up Channels for Postinfarct Ventricular Tachycardia Ablation", PACE - PACING AND CLINICAL ELECTROPHYSIOLOGY., vol. 35, no. 7, 31 mai 2012 (2012-05-31), pages 897-904, XP055243046, US ISSN: 0147-8389, DOI: 10.1111/j.1540-8159.2012.03429.x
- Mauricio Moreno ET AL: "Review Article Pacemapping", Indian Pacing and Electrophysiology, 1 janvier 2005 (2005-01-01), pages 35-42, XP055243362, Extrait de l'Internet: URL:http://www.ncbi.nlm.nih.gov/pmc/articl es/PMC1502070/pdf/ipej050035-00.pdf [extrait le 2016-01-20]
- CORINNA B. BRUNCKHORST ET AL: "Identification of the Ventricular Tachycardia Isthmus After Infarction by Pace Mapping", CIRCULATION, vol. 110, no. 6, 10 August 2004 (2004-08-10), pages 652-659, XP055661004, US ISSN: 0009-7322, DOI: 10.1161/01.CIR.0000138107.11518.AF

**Description**

La présente invention se rapporte à un procédé et un système d'identification d'un isthme dans une cartographie tridimensionnelle. Elle trouve une application particulièrement intéressante dans le domaine des troubles du rythme cardiaque, lors d'une tachycardie ventriculaire par exemple.

Depuis le début des années 1990 et l'utilisation de l'énergie de radiofréquence pour l'ablation par voie endocavitaire des troubles du rythme rapide, les indications de l'ablation se sont progressivement étendues à l'ensemble des troubles du rythme.

En effet, les rythmologues interventionnels prennent en charge des troubles du rythme de plus en plus complexe quant à leur mécanisme et à cette première dimension s'ajoute la nécessité d'une représentation précise dans l'espace du positionnement du cathéter d'ablation et finalement l'intégration des données spatiales et temporelles pour identifier le substrat arythmogène. Des systèmes de cartographie 3D associés à de l'imagerie par résonance magnétique (IRM) constituent un outil très important pour le rythmologue interventionnel. Ces systèmes représentent une aide considérable pour identifier les substrats arythmogènes complexes qui nécessitent des ablations « sur mesure » pour un patient donné.

L'aide apportée par ses systèmes intéresse non seulement la compréhension et le repérage dans l'espace du substrat arythmogène, mais aussi le positionnement précis du cathéter d'ablation. Un autre élément du progrès concerne la réduction du temps d'exposition aux rayons X, à la fois pour le patient mais également pour l'équipe d'électrophysiologie, car ces systèmes n'emploient pas de rayons X dans leur technologie propre.

En fonctionnement normal, les oreillettes du coeur imposent le rythme de battement aux ventricules. Ces derniers reçoivent le sang des oreillettes et se contractent de façon synchronisée pour éjecter le sang soit vers les poumons soit vers les autres organes du corps. Ce sont des impulsions électriques au niveau des oreillettes qui permettent cette contraction. Une tachycardie ventriculaire survient lorsque l'impulsion électrique prend naissance au niveau du muscle d'un des deux ventricules et non pas des oreillettes. La tachycardie ventriculaire (TV) est une arythmie rapide, c'est-à-dire une accélération très rapide du coeur au-delà de 180 battements par minute par exemple. Le coeur ne parvient plus à se remplir, la pompe cardiaque bat alors dans le vide. Ce mauvais fonctionnement peut dégénérer en une autre arythmie, la fibrillation ventriculaire qui peut conduire à un arrêt cardiaque si elle n'est pas traitée avec succès rapidement.

On considère que trois mécanismes fondamentaux peuvent être à l'origine des tachycardies ventriculaires :

- phénomènes de dépolarisation diastolique,
- phénomènes de ré-excitation focale, et
- phénomènes de réentrée, ce dernier étant le plus fréquent.

Le concept de réentrée nécessite la présence d'une zone à conduction lente.

D'une façon générale, le diagnostic est établi à l'aide d'un électrocardiogramme (ECG) enregistré pendant la tachycardie ventriculaire. L'ECG a pour but de recueillir sur plusieurs dérivations (couples d'électrodes) de façon simultanée, l'activité électrique globale du coeur (vecteur cardiaque) : propagation dans le temps et dans l'espace.

Les dérivations correspondent aux couples d'électrodes présentes lors de l'enregistrement. Chaque dérivation donne une image unidirectionnelle du vecteur d'activation cardiaque. Cette image correspond à la projection du vecteur sur la dérivation.

Un ECG standard enregistre l'activité cardiaque sur 12 à 18 dérivations, qui se répartissent en deux catégories :

- Dérivations périphériques et
- Dérivations précordiales.

Le tracé doit comporter les 12 dérivations principales au minimum, c'est-à-dire, dans l'ordre : les trois dérivations standard (D I, D II, D III), les trois dérivations unipolaires des membres (aVR, aVL, aVF), et les six dérivations précordiales de V1 à V6.

Un ECG à 12 dérivations est un ensemble de 12 courbes présentant chacun des formes particulières telles une bosse pour une onde P ou une variation rapide pour un complexe QRS.

Un complexe QRS correspond à la dépolarisation des ventricules. Il a une durée moyenne d'environ 0,08sec.

En ce qui concerne les traitements préventifs, on peut mettre en place un défibrillateur automatique implanté dans le patient de façon à générer des impulsions électriques en cas de début d'arythmie.

On peut également mettre en place un traitement médicamenteux pour réduire le nombre de crises.

Enfin, il est possible de réaliser une ablation par radiofréquence lorsqu'il s'agit d'une arythmie liée au mécanisme de réentrée. Dans ce cas il s'agit d'identifier la zone de faible conduction qui sera détruite, brûlée de façon à créer un barrage au circuit intraventriculaire formant un substrat arythmogène et contenant cette zone de faible conduction. Ce substrat arythmogène, également appelé isthme, peut être déterminé au cours d'une exploration électrophysiologique, le patient étant en tachycardie ventriculaire. Il peut être cautérisé et circonscrit par une sonde dont l'extrémité est chauffée par un courant de radio fréquence. L'examen est aidé par un système de cartographie tridimensionnelle du coeur. Cette technique a une bonne efficacité mais le risque de récidive n'est pas éliminé et ne permet pas, en règle, de se passer de l'implantation d'un défibrillateur. On connaît une telle technique d'ablation d'isthme post infarctus dans laquelle des ECG de surface sont réalisés puis comparés à un ECG de référence enregistré lors d'une tachycardie ventriculaire. Une telle technique est décrite dans le document « Localizing the critical isthmus of postinfarct ventricular tachycardia: the value of pace-mapping during sinus rhythm » , de Chillou et al, Heart Rhythm ; 2014 Feb;11(2):175-81.

On connaît le document T. DICKFELD ET AL : 'MRI-Guided Ventricular Tachycardia Ablation : intégration of Late Gadolinium-Enhanced 3D Scar in Patients With Implantable Cardioverter-Defribillators', CIRCULATION. ARRYHTMIA AND ELECTROPHYSIOLOGY, vol. 4, n° 2, 26 janvier 2011 (2011-01-26), pages 172-184. Ce document divulgue l'ablation des tachycardies ventriculaires assistée par IRM cardiaque. La technique de 'pace-mapping' par la méthode d'ECG utilisant un ECG de référence y est mentionnée. U

On connaît le document Pr. De chillou : 'Catheter ablation of post-infract VT Background', Institut Lorrain du coeur et des vaisseaux - University Hospital Nancy - France, 30 janvier 2014 (2014-01-30), pages 1-97, extrait de l'internet : http:// sfcardio.fr/sites/defaut/files/pdf/Chillou-a565.pdf . Ce document décrit l'utilisation de la technique de 'pace-mapping' par la méthode d'ECG utilisant un ECG de référence pour identifier l'isthme d'une tachycardie ventriculaire. U

On connaît le document W. G. STEVENSON ET AL. : 'Catheter Ablation for Ventricular Tachycardia', CIRCULATION, vol. 115, n° 21, 14 mai 2007 (2007-05-14). Ce document est un document sur l'état de l'art de l'ablation des tachycardies ventriculaires. Il divulgue la technique de 'pace-mapping' par la méthode d'ECG utilisant un ECG de référence avec mesures itératives des S-QRS. U

On connaît le document CHRISTIAN DE CHILLOU ET AL. : 'Showing up Channels for Postinfarct Ventricular tachycardia Ablation', PACE - PACING AND CLINICAL ELECTROPHYSIOLOGY., vol. 35, n° 7, 31 mai 2012 (2012-05-31), pages 897-904. Ce document divulgue le même contenu que le précédent. U

On connaît le document Mauricio Moreno ET AL : 'Review Article Pacemapping', Idian Pacing and Electrophysiology, 1 janvier 2005 (2005-01-01), pages 35-42. Ce document est un article de revue sur les techniques 'pace-mapping', il y est fait mention de la cartographie par la méthode ECG utilisant un ECG de référence. U

L'invention est exposée dans le jeu de revendications annexé.

La présente invention a pour but un nouveau procédé de détermination de l'isthme de façon rapide.

Un autre but de l'invention est une identification de l'isthme de façon complètement préventive même pour des patients qui ne sont pas capables de supporter une tachycardie ventriculaire, notamment provoquée.

On atteint au moins l'un des objectifs précités avec un procédé d'identification d'un isthme dans une cartographie tridimensionnelle d'une cavité cardiaque, au moyen d'une unité de traitement configurée pour réaliser les étapes suivantes :

a) corrélation entre un ensemble de points stimulés de la cavité cardiaque, chaque point stimulé étant représenté par un ensemble de signaux obtenus suite à une électrocardiographie (ECG) de surface, hors tachycardie ventriculaire, en rythme sinusal par exemple,
b) identification d'une ligne de partage des eaux à partir des résultats de corrélation ci-dessus et des coordonnées 3D des points stimulés dans la cartographie 3D,
c) détermination de l'isthme à partir d'un couloir 3D sensiblement transverse à la ligne de partage des eaux.

L'invention concerne un procédé d'identification d'une ligne de partage des eaux dans une cartographie tridimensionnelle d'une cavité cardiaque, au moyen d'une unité de traitement configurée pour réaliser les étapes suivantes :

a) corrélation entre un ensemble de points stimulés de la cavité cardiaque, chaque point stimulé étant représenté par un ensemble de signaux obtenus suite à une électrocardiographie (ECG) de surface, hors tachycardie ventriculaire, en rythme sinusal par exemple,
b) identification d'une ligne de partage des eaux à partir des résultats de corrélation ci-dessus et des coordonnées 3D des points stimulés dans la cartographie 3D.

L'expression « hors tachycardie ventriculaire » est une expression claire pour l'homme du métier qui signifie que l'ECG est acquis selon un rythme de base qui n'est pas la tachycardie ventriculaire (TV). Il est connu de l'homme du métier que le rythme hors TV peut être un rythme sinusal dans la plupart du temps, mais cela peut aussi être en fibrillation atriale, en rythme électro-entraîné par un pacemaker...

La ligne de partage des eaux correspond à variation brutale des corrélations entre proches voisins.

Avec le procédé selon l'invention, on n'utilise pas une électrocardiographie de référence qui est généralement une électrocardiographie de tachycardie ventriculaire, ce dernier a pour inconvénient d'être long à réaliser et de placer le patient dans des conditions à risques.

La corrélation entre les différents points permet de mettre en évidence une variation brusque géographique entre niveaux de corrélation, cette zone géographique de variation brusque correspond à une zone de très faible conduction électrique.

Selon l'invention, les points stimulés sont des endroits du coeur d'un patient qui sont stimulés de façon à recueillir une électrocardiographie. Ces points peuvent être choisis de façon aléatoire dans tout le volume de la cavité cardiaque, mais ils peuvent également être déterminés de façon méthodique.

Selon une caractéristique avantageuse de l'invention, préalablement à l'étape a), on réalise une étape de constitution de plusieurs volumes qui se chevauchent dans la cartographie 3D, ces volumes contenant l'ensemble des points stimulés, l'étape a) étant réalisée entre des points stimulés de chacun des volumes.

On constitue ainsi des familles de points. Dans chaque famille on réalise des calculs de coefficient de corrélation de chaque point par rapport à tous les autres ou de chaque point par rapport à ces proches voisins uniquement. Un point peut faire partie de plusieurs volumes ou familles, on assure ainsi une continuité dans toute la cavité cardiaque.

Selon un mode de réalisation avantageux de l'invention, pour gagner du temps lors de la mise en oeuvre, on ne stimule pas équitablement l'ensemble de la cavité cardiaque. Selon l'invention, on peut de préférence réaliser au moins une itération des étapes a) et b) ; à chaque itération, on ajoute de nouveaux points stimulés à proximité de la ligne de partage des eaux identifié à l'itération précédente. De cette façon, on réalise un maximum de calcul de coefficient de corrélation autour de la ligne de partage des eaux qui est la zone qui permet d'identifier précisément l'isthme. Les autres zones peuvent contenir très peu de points stimulés. La réalisation des itérations permet de réaliser d'abord des identifications grossières jusqu'à des identifications de plus en plus précise ; le critère d'arrêt des itérations pouvant être la durée, nombre de points stimulés ou bien un nombre prédéterminé d'itérations.

De préférence, lesdits signaux peuvent correspondre aux 12 dérivations d'une ECG de surface.

Selon un mode de réalisation avantageux de l'invention, la corrélation est réalisée sur des complexes QRS issus desdits signaux. Dans ce cas une première peut être l'identification des complexes QRS dans les signaux. Cela permet d'améliorer la rapidité des calculs et par conséquent la rapidité de tout le processus d'identification de l'isthme.

En particulier, la corrélation peut être mise en oeuvre selon l'algorithme BARD dit de «template matching» ou diagramme de correspondance. Il s'agit d'une méthode notamment décrite dans le document « Quantitative Comparison of Spontaneous and Paced 12-Lead Electrocardiogram During Right Ventricular Outflow Tract Ventricular Tachycardia », Gerstenfeld EP et al. J Am Coll Cardiol 2003;41:2046-53.

Il s'agit d'effectuer la comparaison de la morphologie des complexes de l'ECG à 12 dérivations. La méthode Bard définit un calcul numérique permettant de comparer par des critères objectifs deux électrocardiogrammes à douze dérivations.

Le coefficient de corrélation CORR est calculé de la façon habituelle. Pour deux formes d'onde complètement à l'opposé le coefficient de corrélation CORR = -1. Pour deux formes d'onde identiques coefficient de corrélation CORR = 1. Deux formes d'ondes avec une morphologie similaire contenant un tiers de la zone de l'autre ont un coefficient de corrélation CORR=1.

Pour comparer les multiples formes d'onde, telles que celles des complexes d'une ECG à 12 dérivations, la formule est:

$$CORR = \frac{\sum_{dérivation\ 1}^{12}[\sum_{i=1}^{n}(X_i - \bar{X}).(Y_i - \bar{Y})]}{\sum_{dérivation\ 1}^{12}\left[\sqrt{\sum_{i=1}^{n}(X_i - \bar{X})^2.\sum_{i=1}^{n}(Y_i - \bar{Y})^2}\right]}$$

où X et Y sont des vecteurs de longueur n représentant les deux signaux à comparer. Le coefficient de corrélation CORR varie généralement de -1 pour forme d'onde complètement opposée à +1 pour des signaux identiques.

Selon une caractéristique de l'invention, l'étape a) comprend en outre une étape d'identification de groupes de points stimulés en fonction du niveau de corrélation entre ces points stimulés.

La première répartition par famille est une répartition uniquement géographique, ici on utilise comme critère le niveau du coefficient de corrélation. Avantageusement, l'identification de groupes est réalisée par affichage sur un écran de visualisation, avec une couleur identique, l'ensemble de points stimulés d'un même groupe. On détermine un code couleur permettant d'afficher un groupe ayant un fort coefficient de corrélation entre eux. Ceci peut permettre d'identifier facilement un isthme de façon visuelle.

Selon un autre aspect de l'invention, il est proposé un système pour identifier un isthme dans une cartographie tridimensionnelle d'une cavité cardiaque, ce système comprenant :

- un dispositif de cartographie comprenant un cathéter dont une extrémité peut être placée à différents endroits de la cavité cardiaque d'un patient, ce dispositif de cartographie étant également relié à des électrodes pour l'élaboration de cartographies 3D ainsi l'élaboration d'ECG à douze dérivations,
- une électrocardiographe pour générer une électrocardiographie en stimulant plusieurs points d'un corps, et
- une unité de traitement configurée pour réaliser les étapes suivantes :

a) corrélation entre un ensemble de points stimulés de la cavité cardiaque, chaque point stimulé étant représenté par un ensemble de signaux obtenus suite à une électrocardiographie (ECG) de surface, hors tachycardie ventriculaire,
b) identification d'une ligne de partage des eaux à partir des résultats de corrélation ci-dessus et des coordonnées 3D des points stimulés dans la cartographie 3D,
c) détermination de l'isthme à partir d'un couloir 3D sensiblement transverse à la ligne de partage des eaux.

Ce système peut avantageusement comprendre en outre un cathéter d'ablation.

D'autres avantages et caractéristiques de l'invention apparaîtront à l'examen de la description détaillée d'un mode de mise en oeuvre nullement limitatif, et des dessins annexés, sur lesquels :

- La figure 1 est une représentation tridimensionnelle d'une cavité cardiaque avec une illustration d'un circuit électrique en cas de tachycardie ventriculaire,
- La figure 2 est une vue schématique simplifiée du circuit électrique réentrant laissant apparaître l'isthme à identifier,
- Les figures 3 à 5 sont des vues schématiques d'un ensemble de points stimulés dans la cavité cardiaque, ces différentes figurent illustrant différentes étapes de corrélation deux à deux entre les différents points stimulés de façon à constituer une carte de densité permettant d'opérer des regroupements entre points stimulés fortement corrélés,
- La figure 6 est une vue schématique illustrant le circuit de tachycardie ventriculaire avec visualisation de la zone à ablater à travers de l'isthme,
- La figure 7 est une vue schématique de deux électrocardiogrammes devant subir une opération de corrélation, et
- La figure 8 est une vue schématique très simplifié d'un système pour la mise en oeuvre du procédé selon l'invention.

Dans l'art antérieur, il était parfois nécessaire de provoquer un épisode de tachycardie ventriculaire de façon à avoir un ECG de référence. Ce dernier était ensuite comparé à d'autres ECG en rythme sinusal (état normal de fonctionnement du coeur). Avec la présente invention, on évite cet épisode déclenché parfois artificiellement. En effet, cela pouvait être complexe pour des patients équipés d'un stimulateur cardiaque ou d'un défibrillateur définitifs.

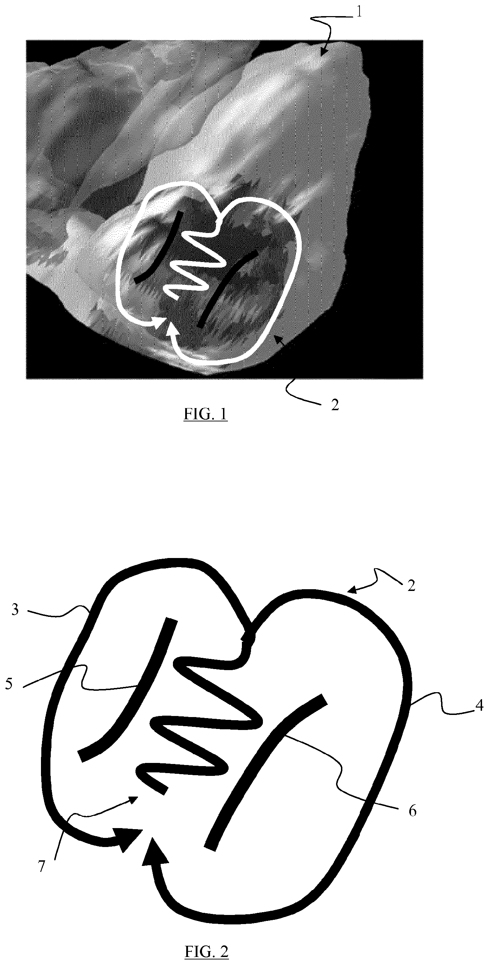

La présente invention fait appel à un système de cartographie 3D permettant d'avoir une représentation tridimensionnelle du coeur comme on peut le voir sur la figure 1. En particulier, on distingue globalement le ventricule gauche 1. Il s'agit d'une cartographie d'amplitude du ventricule gauche d'un patient qui présente une séquelle d'infarctus antérieur. Les zones saines apparaissent globalement en dehors du circuit 2 dessiné sur la cartographie et la séquelle d'infarctus globalement à l'intérieur de ce circuit 2.

Le circuit 2 est plus clairement représenté sur la figure 2. Ce circuit représente le cheminement d'un front de dépolarisation au cours d'un épisode de tachycardie. Toutes les tachycardies ventriculaires peuvent être représentées par un circuit 2 réentrant en forme d'une double boucles formant un « 8 », les deux boucles 3 et 4 étant le siège d'un front de dépolarisation circulant dans des sens opposés autour de barrières 5 et 6 délimitant l'isthme. L'isthme est la zone centrale 7 qui forme le substrat arythmogène de l'arythmie cartographiée.

Le traitement de la tachycardie ventriculaire revient à réaliser une ablation de l'isthme. Plus précisément il s'agit de brûler par onde radiofréquence une partie de l'isthme de façon à réaliser une coupure de celui-ci et éviter ainsi la propagation de l'onde de dépolarisation.

L'invention est notamment remarquable par le fait qu'on détermine la zone d'ablation sans avoir recours à un ECG d'une tachycardie ventriculaire préalable.

Pour ce faire, on utilise un cathéter pour stimuler pendant une dizaine de secondes plusieurs points de la cavité cardiaque. A chaque point stimulé, on recueille un ECG de douze dérivations. La figure 2 illustre une répartition aléatoire de ces points. A priori le circuit 2 qui est représenté sue les figures 2 à 6 n'est pas connu. Il est simplement représenté par souci de compréhension. La répartition des points peut être aléatoire mais elle peut également être obtenue de façon méthodique, notamment prédéterminée, de façon par exemple à couvrir équitablement une surface ou un volume. La répartition peut donc être homogène ou bien définie en fonction de critères physiologiques.

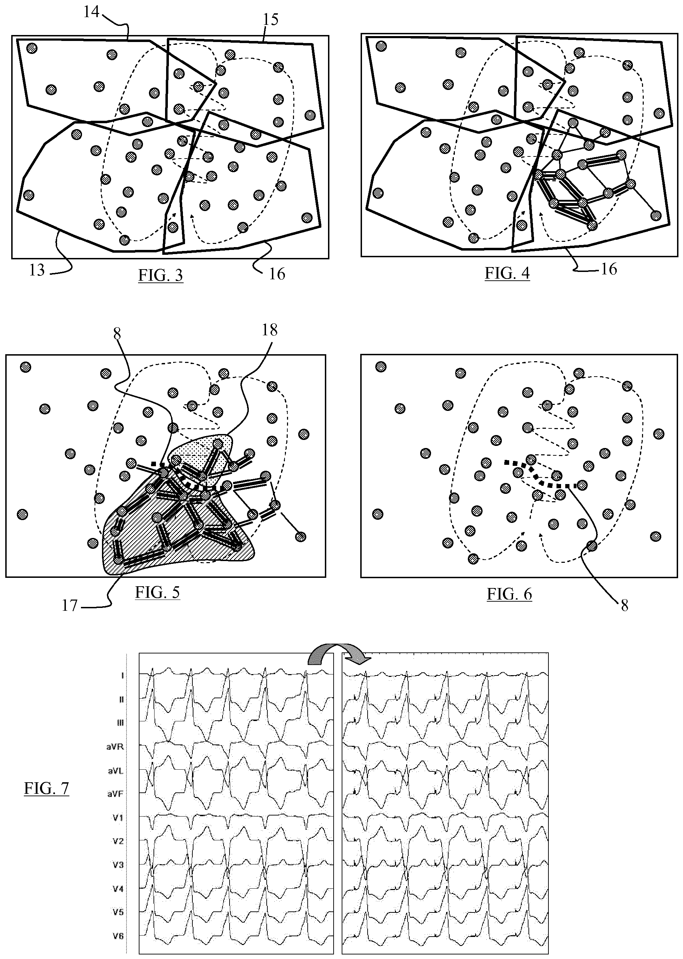

Dans un deuxième temps on regroupe plusieurs points géographiquement proches. Ces groupes ainsi constitués peuvent se chevaucher de sorte qu'un point peut appartenir à plusieurs groupes. Chaque groupe est une famille de points qui sont géographiquement proches entre eux. Sur la figure 3, on distingue par exemple quatre familles 13, 14, 15 et 16.

On détermine un coefficient de corrélation pour chaque couple d'une famille. Pour cela, on utilise la méthode Bard en tenant compte des douze dérivations associées à chaque point stimulé. On établit ainsi une cartographie de densités des liens entre différents points stimulés comme on peut le voir très schématiquement sur la figure 4 en ce qui concerne la famille 16. Dans chaque famille, on peut mettre en place des techniques permettant de proche en proche de déterminer les coefficients de corrélation sans obligatoirement calculer ce coefficient pour tous les couples de la famille.

Sur la figure 7 on voit deux ensembles de signaux de deux électrocardiogrammes obtenus après stimulation de deux points en rythme sinusal. Contrairement à l'art antérieur, on n'utilise pas ici un ECG de référence issu d'une tachycardie ventriculaire préalable. Les ECG sont comparés les uns aux autres, de préférence par famille ou bien de façon générale.

On identifie ensuite des points ayant sensiblement la même densité, c'est-à-dire des points qui sont fortement liés entre eux indépendamment des familles constituées plus haut. On forme ainsi des groupes de points fortement liés entre eux comme on le voit sur la figure 5 pour les groupes 17 et 18.

Sur la figure 5, on identifie une ligne de partage des eaux 8, c'est-à-dire l'endroit où l'on constate une chute brutale, un véritable décalage entre les deux groupes 17 et 18. On peut représenter cela comme une « falaise » qui sépare deux groupes voisins. Cela se fait par la technique de ligne de partage des eaux ou par toute autre technique permettant de détecter deux groupes voisins ayant le plus faible lien entre eux. Cette falaise correspond à une zone de faible conduction.

Idéalement, la ligne de partage des eaux 8 est la zone à brûler. Sur la figure 6, l'isthme est la zone sensiblement perpendiculaire à la ligne de partage des eaux 8. L'ablation permettant de couper l'isthme peut également être réalisée à tout autre endroit différent de la ligne de partage des eaux. Lorsque les points stimulés ne sont pas suffisants pour calculer correctement la ligne de partage des eaux, on acquiert d'autres points stimulés autour de la zone ou des zones pressenties de façon à calculer avec certitude la ligne de partage des eaux.

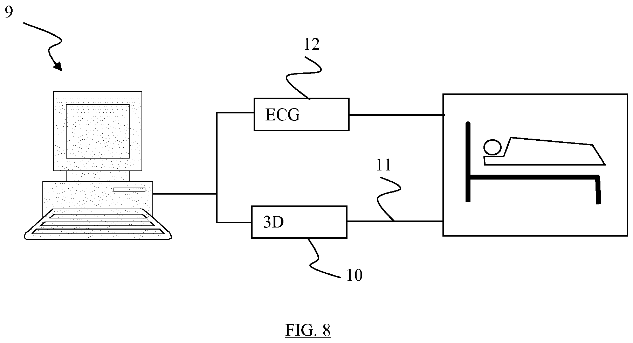

La figure 8 illustre de façon schématique un système permettant de mettre en application la présente invention. On distingue une unité de traitement 9 équipé d'au moins un microprocesseur, des espaces mémoires, des cartes de communication à des périphériques externes, des composants d'entrée/sortie et d'un moyen d'affichage. Cette unité de traitement est reliée à un dispositif de cartographie 10 comprenant un cathéter 11 dont une extrémité peut être placée à différents endroits de la cavité cardiaque d'un patient. Le dispositif de cartographie 10 est également relié à des électrodes (non représentées) pour l'élaboration de cartographies 3D ainsi l'élaboration d'ECG à douze dérivations.

Le cathéter permet de stimuler tout point de la cavité cardiaque. Il permet également de réaliser une ablation par onde radiofréquence.

Pour réaliser une cartographie 3D, on utilise des sources d'émission électromagnétique sont placées aux sommets d'un cadre triangulaire, lui-même positionné sous la table d'examen sur laquelle le patient est installé. L'un des capteurs électromagnétiques (la référence spatiale) est incorporé dans un patch cutané positionné sous fluoroscopie en regard de l'ombre cardiaque au niveau du dos du patient. L'autre capteur est incorporé au niveau de l'extrémité distale du cathéter d'ablation que l'on va déplacer en différents points au niveau de la surface endocardique de la cavité cardiaque qui va être cartographiée au cours de l'examen. Le déplacement de ce cathéter peut être observé en temps réel sur un écran de contrôle. À chaque nouvelle position du cathéter dans une cavité cardiaque donnée, on peut faire l'acquisition de cette position sous la forme d'un point qui apparaîtra sur l'écran de contrôle. Les points ainsi acquis seront automatiquement reliés entre eux par le programme informatique qui créera une surface virtuelle entre les différents points et, avec l'accumulation de ceux-ci on obtiendra une forme géométrique tridimensionnelle qui épousera exactement les contours endocardiques de la cavité cardiaque cartographiée. Le cathéter est muni d'électrodes permettant un recueil du signal endocavitaire bipolaire et unipolaire au niveau de chacun des points qui forment la reconstruction virtuelle de la cavité cardiaque. Il est donc possible, à l'aide d'un codage couleur corrélé à l'amplitude bipolaire ou unipolaire du signal recueilli, d'obtenir une cartographie d'amplitude de la cavité cardiaque examinée.

Sur la figure 8, on distingue également une baie d'électrophysiologie 12 permettant d'élaborer des ECG à douze dérivations.

Bien sûr, l'invention n'est pas limitée aux exemples qui viennent d'être décrits et de nombreux aménagements peuvent être apportés à ces exemples sans sortir du cadre de l'invention. La présente invention peut comprendre une unité de traitement recevant en entrée une cartographie 3D d'une cavité cardiaque, complétée éventuellement d'une imagerie par résonance magnétique, ainsi que des ECG en rythme sinusal d'un ensemble de points stimulés. La sortie de l'unité de traitement peut être une image de la cavité cardiaque sur laquelle est affichée en superposition la ligne e partage des eaux. Cette ligne de partage des eaux peut être représentée sous la forme d'un ensemble de coordonnées spatiales utilisables pour l'ablation.

Le procédé selon l'invention permet avantageusement d'identifier l'isthme d'une tachycardie ventriculaire post-infarctus indépendamment de la disponibilité d'un ECG à 12 dérivations lors de la tachycardie ventriculaire. Il est désormais possible de réaliser des traitements préventifs par ablation par radiofréquence au moyen d'un cathéter pour un grand nombre de patients post-infarctus.

**Revendications**

1. Procédé d'identification d'un isthme dans une cartographie tridimensionnelle d'une cavité cardiaque, au moyen d'une unité de traitement (9) configurée pour réaliser les étapes suivantes :

   a) corrélation entre un ensemble de points stimulés de la cavité cardiaque, chaque point stimulé étant représenté par un ensemble de signaux obtenus suite à une électrocardiographie (ECG) de surface, hors tachycardie ventriculaire,
   b) identification d'une ligne de partage des eaux (8) à partir des résultats de corrélation ci-dessus et des coordonnées 3D des points stimulés dans la cartographie 3D,
   c) détermination de l'isthme (7) à partir d'un couloir 3D sensiblement transverse à la ligne de partage des eaux (8).

2. Procédé selon la revendication 1, **caractérisé en ce que**, préalablement à l'étape a), on réalise une étape de constitution de plusieurs volumes qui se chevauchent dans la cartographie 3D, ces volumes contenant l'ensemble des points stimulés, l'étape a) étant réalisée entre des points stimulés de chacun des volumes.

3. Procédé selon la revendication 1 ou 2, **caractérisé en ce qu'**on réalise au moins une itération des étapes a) et b) ; à chaque itération, on ajoute de nouveaux points stimulés à proximité de la ligne de partage des eaux identifié à l'itération précédente.

4. Procédé selon l'une quelconque des revendications précédentes, **caractérisé en ce que** lesdits signaux correspondent aux 12 dérivations d'une ECG de surface.

5. Procédé selon la revendication 4, **caractérisé en ce que** la corrélation est réalisée sur des complexes QRS issus desdits signaux.

6. Procédé selon la revendication 5, **caractérisé en ce que** la corrélation est mise en oeuvre selon l'algorithme BARD dit de «template matching».

7. Procédé selon l'une quelconque des revendications précédentes, **caractérisé en ce que** l'étape a) comprend en outre une étape d'identification de groupes de points stimulés en fonction du niveau de corrélation entre ces points stimulés.

8. Procédé selon la revendication 7, **caractérisé en ce que** l'identification de groupes est réalisée par affichage sur un écran de visualisation, avec une couleur identique, l'ensemble de points stimulés d'un même groupe.

9. Système pour identifier un isthme dans une cartographie tridimensionnelle d'une cavité cardiaque, ce système comprenant :

   - un dispositif de cartographie (10) comprenant un cathéter (11) dont une extrémité peut être placée à différents endroits de la cavité cardiaque d'un patient, ce dispositif de cartographie (10) étant également relié à des électrodes pour l'élaboration de cartographies 3D ainsi l'élaboration d'ECG à douze dérivations,
   - un électrocardiographe pour générer une électrocardiographie en stimulant plusieurs points d'un corps, et
   - une unité de traitement (9) configurée pour réaliser les étapes suivantes :

   a) corrélation entre un ensemble de points stimulés de la cavité cardiaque, chaque point stimulé étant représenté par un ensemble de signaux obtenus suite à une électrocardiographie (ECG) de surface, hors tachycardie ventriculaire,
   b) identification d'une ligne de partage des eaux (8) à partir des résultats de corrélation ci-dessus et des coordonnées 3D des points stimulés dans la cartographie 3D,
   c) détermination de l'isthme (7) à partir d'un couloir 3D sensiblement transverse à la ligne de partage des eaux (8).

10. Système selon la revendication 9, **caractérisé en ce qu'**il comprend en outre un cathéter d'ablation (11).

**Patentansprüche**

1. Verfahren zur Identifizierung eines Isthmus in einer dreidimensionalen Kartierung einer Herzkammer mittels einer Verarbeitungseinheit (9), die dazu ausgelegt ist, die folgenden Schritte auszuführen:

   a) Korrelationsberechnung zwischen einem Satz von stimulierten Punkten der Herzkammer, wobei jeder stimulierte Punkt durch einen Satz von Signalen dargestellt wird, die infolge einer Oberflächen-Elektrokardiographie (EKG) erhalten werden, ausgenommen ventrikuläre Tachykardie,
   b) Identifizieren einer Wasserscheide (8) aus den obigen Korrelationsergebnissen und den 3D-Koordinaten der stimulierten Punkte in der 3D-Kartierung,
   c) Ermitteln des Isthmus (7) anhand eines 3D-Korridors, der im Wesentlichen quer zur Wasserscheide (8) verläuft.

2. Verfahren nach Anspruch 1, **dadurch gekennzeichnet, dass** vor Schritt a) ein Schritt des Bildens mehrerer sich überlappender Volumina in der 3D-Kartierung ausgeführt wird, wobei diese Volumina die Gesamtheit der stimulierten Punkte enthalten, wobei Schritt a) zwischen stimulierten Punkten in jedem der Volumina ausgeführt wird.

3. Verfahren nach Anspruch 1 oder 2, **dadurch gekennzeichnet, dass** mindestens eine Iteration der Schritte a) und b) erfolgt; wobei bei jeder Iteration neue stimulierte Punkte in der Nähe der Wasserscheide hinzugefügt werden, die in der vorherigen Iteration identifiziert wurde.

4. Verfahren nach einem der vorhergehenden Ansprüche, **dadurch gekennzeichnet, dass** die Signale den 12 Ableitungen eines Oberflächen-EKGs entsprechen.

5. Verfahren nach Anspruch 4, **dadurch gekennzeichnet, dass** die Korrelation an QRS-Komplexen aus den Signalen erfolgt.

6. Verfahren nach Anspruch 5, **dadurch gekennzeichnet, dass** die Korrelation nach dem Template-Matching-Algorithmus BARD erfolgt.

7. Verfahren nach einem der vorhergehenden Ansprüche, **dadurch gekennzeichnet, dass** Schritt a) ferner einen Schritt der Identifizierung von Gruppen von stimulierten Punkten in Abhängigkeit vom Grad der Korrelation zwischen diesen stimulierten Punkten umfasst.

8. Verfahren nach Anspruch 7, **dadurch gekennzeichnet, dass** die Identifizierung von Gruppen dadurch erfolgt, dass die Gesamtheit der stimulierten Punkte einer gleichen Gruppe auf einem Bildschirm mit einer identischen Farbe angezeigt wird.

9. System zur Identifizierung eines Isthmus in einer dreidimensionalen Kartierung einer Herzkammer, wobei das System das Folgende umfasst:

   - eine Kartierungsvorrichtung (10) mit einem Katheter (11), von dem ein Ende an verschiedenen Stellen der Herzkammer eines Patienten platziert werden kann, wobei diese Kartierungsvorrichtung (10) auch mit Elektroden zur Erstellung von 3D-Kartierungen sowie zur Erstellung von EKGs mit zwölf Ableitungen verbunden ist,
   - eine Elektrokardiographie-Einheit zum Erzeugen einer Elektrokardiographie durch Stimulieren mehrerer Punkte eines Körpers, und
   - eine Verarbeitungseinheit (9), die dazu ausgelegt ist, die folgenden Schritte auszuführen:

      a) Korrelationsberechnung zwischen einem Satz von stimulierten Punkten der Herzkammer, wobei jeder stimulierte Punkt durch einen Satz von Signalen dargestellt wird, die infolge einer Oberflächen-Elektrokardiographie (EKG), ausgenommen ventrikuläre Tachykardie, erhalten werden,
      b) Identifizieren einer Wasserscheide (8) aus den obigen Korrelationsergebnissen und den 3D-Koordinaten der stimulierten Punkte in der 3D-Kartierung,
      c) Ermitteln des Isthmus (7) anhand eines 3D-Korridors, der im Wesentlichen quer zur Wasserscheide (8) verläuft.

10. System nach Anspruch 9, **dadurch gekennzeichnet, dass** es ferner einen Ablationskatheter (11) umfasst.

**Claims**

1. Method for identifying an isthmus in a three-dimensional mapping of a cardiac cavity, by means of a processing unit (9) configured to carry out the following steps:

    a) correlation between a set of stimulated points of the cardiac cavity, each stimulated point being represented by a set of signals obtained following surface electrocardiography (ECG), excluding ventricular tachycardia,
    b) identifying a watershed line (8) based on the above correlation results and the 3D coordinates of the stimulated points in the 3D mapping,
    c) determining the isthmus (7) based on a 3D corridor substantially transverse to the watershed line (8).

2. Method according to claim 1, **characterized in that**, prior to step a), a step of constituting several volumes which overlap in the 3D mapping is carried out, these volumes containing the set of stimulated points, step a) being carried out between stimulated points of each of the volumes.

3. Method according to claim 1 or 2, **characterized in that** at least one iteration of steps a) and b) is carried out; at each iteration, new stimulated points are added close to the watershed line identified in the preceding iteration.

4. Method according to any one of the preceding claims, **characterized in that** said signals correspond to the 12 leads of a surface ECG.

5. Method according to claim 4, **characterized in that** the correlation is carried out on QRS complexes originating from said signals.

6. Method according to claim 5, **characterized in that** the correlation is implemented according to the BARD algorithm called "template matching".

7. Method according to any one of the preceding claims, **characterized in that** step a) comprises moreover a step for identifying groups of stimulated points as a function of the level of correlation between these stimulated points.

8. Method according to claim 7, **characterized in that** the groups are identified by displaying on a display screen, with an identical colour, the set of stimulated points of one and the same group.

9. System for identifying an isthmus in a three-dimensional mapping of a cardiac cavity, this system comprising:

    - a mapping device (10) comprising a catheter (11) one end of which can be placed at different sites of the cardiac cavity of a patient, this mapping device (10) being also connected to electrodes for producing 3D mappings and thus producing 12-lead ECGs,
    - an electrocardiograph for generating an electrocardiography by stimulating several points of a body, and
    - a processing unit (9) configured to carry out the following steps:

        a) correlation between a set of stimulated points of the cardiac cavity, each stimulated point being represented by a set of signals obtained following surface electrocardiography (ECG), excluding ventricular tachycardia,
        b) identifying a watershed line based (8) following the above correlation results and the 3D coordinates of the stimulated points in the 3D mapping,
        c) determining the isthmus (7) based on a 3D corridor substantially transverse to the watershed line (8).

10. System according to claim 9, **characterized in that** it comprises moreover an ablation catheter (11).

FIG. 1

FIG. 2

FIG. 3

FIG. 4

FIG. 5

FIG. 6

FIG. 7

FIG. 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **CHILLOU et al.** Localizing the critical isthmus of postinfarct ventricular tachycardia: the value of pace-mapping during sinus rhythm. *Heart Rhythm,* Février 2014, vol. 11 (2), 175-81
- **T. DICKFELD et al.** MRI-Guided Ventricular Tachycardia Ablation : intégration of Late Gadolinium-Enhanced 3D Scar in Patients With Implantable Cardioverter-Defibrillators. *CIRCULATION. ARRYHTMIA AND ELECTROPHYSIOLOGY,* 26 Janvier 2011, vol. 4 (2), 172-184
- **DE CHILLOU.** Catheter ablation of post-infract VT Background. Institut Lorrain du coeur et des vaisseaux - University Hospital Nancy, 30 Janvier 2014, 1-97
- **W. G. STEVENSON et al.** Catheter Ablation for Ventricular Tachycardia. *CIRCULATION,* 14 Mai 2007, vol. 115 (21
- **CHRISTIAN DE CHILLOU et al.** Showing up Channels for Postinfarct Ventricular tachycardia Ablation. *PACE - PACING AND CLINICAL ELECTROPHYSIOLOGY,* 31 Mai 2012, vol. 35 (7), 897-904
- **MAURICIO MORENO et al.** Review Article Pacemapping. *Idian Pacing and Electrophysiology,* 01 Janvier 2005, 35-42
- **GERSTENFELD EP et al.** Quantitative Comparison of Spontaneous and Paced 12-Lead Electrocardiogram During Right Ventricular Outflow Tract Ventricular Tachycardia. *J Am Coll Cardiol,* 2003, vol. 41, 2046-53